# EUROPEAN PATENT SPECIFICATION

(11) **EP 2 917 317 B1**
(45) Date of publication and mention of the grant of the patent: **09.08.2017**
(21) Application number: 13799496.8
(22) Date of filing: 12.11.2013
(51) Int. Cl.: C11B 9/00

(54) **IMPROVEMENTS IN OR RELATING TO ORGANIC COMPOUNDS**
VERBESSERUNGEN AN ODER IM ZUSAMMENHANG MIT ORGANISCHEN VERBINDUNGEN
AMÉLIORATIONS APPORTÉES À DES COMPOSÉS ORGANIQUES OU S'Y RAPPORTANT

(30) Priority: 12.11.2012 GB 201220341
(43) Date of publication of application: 16.09.2015
(73) Proprietor: Givaudan SA, 1214 Vernier (CH)
(72) Inventor: FLACHSMANN, Felix, CH-8600 Duebendorf (CH); NATSCH, Andreas, CH-8707 Uetikon (CH); BACHMANN, Jean-Pierre, CH-8820 Wädenswil (CH)
(74) Representative: Simmons, John Murray
(86) International application number: PCT/EP2013/073560
(87) International publication number: WO 2014/072519

(56) References cited:
- EP-B1- 0 672 746
- US-A1- 2005 282 728
- US-B1- 6 395 260
- I. R. WHITE ET AL: "Isoeugenol is an important contact allergen: can it be safely replaced with isoeugenyl acetate?", CONTACT DERMATITIS, vol. 41, no. 5, 1 November 1999 (1999-11-01), pages 272-275, XP055100957, ISSN: 0105-1873, DOI: 10.1111/j.1600-0536.1999.tb06160.x

## Description

The present invention is concerned with fragrance ingredients and with fragrance preparations, for imparting desirable odour notes to consumer products, in particular it is concerned with fragrance oils that can be used as replacements in whole or in part for the allergy-disputed compound iso-eugenol. Isoeugenol is a widely used ingredient in perfumery. It is valued for its floral and floral-spicy accords such as carnation and lily. In principle, it can be used over a wide range of doses, for example between 0.1 to 10 % by weight. However, owing to concerns regarding its potential for eliciting skin allergies, for most practical purposes its use is limited to 200 ppm in consumer products, corresponding to a use of about 0.2 to 2 % in a fragrance oil. Contact Dermatitis, 1999, 41, 272-275 describes the use of isoeugenyl acetate as a non-allergenic replacement for iso-eugenol.

Accordingly, there is a need to provide ingredients that can be used in fragrance preparations as a replacement for isoeugenol or which use in fragrance preparations can substantially reduce the amount of isoeugenol used in such preparations.

Fragrance ingredients exhibiting floral-spicy notes as isoeugenol include vanillin and methyl diantilis. However, the use of each of these ingredients is not without its problems. For example, vanillin exhibits a tendency towards discolouration when used in fragrance preparations across a range of consumer bases. Methyl diantilis on the other hand is difficult to use in perfume oils owing to its high water solubility and cannot be formulated easily in sufficiently high amounts for most consumer applications as a result. Besides this, methyl diantilis usage levels above 1 or 2 % in fragrance oil has somewhat undesirable hedonic profile, in particular, it begins to impose undesired guaiacol or phenolic notes. There remains a need for fragrance ingredients that can be used in fragrance oils either as a partial or complete replacement for isoeugenol, which ingredients are non-allergenic and exhibit good colour stability in consumer product bases upon storage under accelerated conditions.

The invention provides in a first aspect a fragrance oil comprising a compound of formula (I) 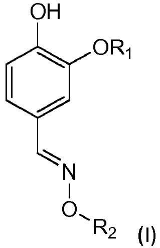 wherein R₁ represents a methyl group or an ethyl group and R₂ represents a methyl or an ethyl group, additionally comprising one or more perfume ingredients, wherein the fragrance oil contains less than 1% by weight of isoeugenol. Particular compounds of the formula (I) are those wherein R₁ is methyl and R₂ is methyl; or wherein R₁is methyl and R₂ is ethyl. The most preferred is the compound in which R₁ is ethyl and R₂ represents a methyl group.

The skilled person will appreciate that compounds of formula (I) can be in the form of E and Z isomers about the carbon-nitrogen double bond. When reference is made herein to a compound of formula (I) it is intended to include the E or Z isomer in pure form as well as mixtures thereof.

Compounds of the formula (I) are known in the art. Indeed, certain compounds of formula (I) and their odour descriptions are disclosed in EP 0 672 746 B1. In particular, 3-ethoxy-4-hydroxy benzaldehyde o-methyl oxime is described as having a vanilla, spicy odour. However, the applicant was unable to find any reference in the literature relating to perfume compositions containing these specific compounds in combination with at least one additional perfume ingredient and consequently the applicant was unable to find any reference to the performance of these compounds in perfume compositions or consumer product containing same. In fact, oxime ethers tend to have much higher odour preception thresholds than their corresponding aldehydes. For example whereas (2E,6Z)-nona-2,6-dienal has a perception threshold of 0.06 ng/l air, its corresponding oxime ether - (1E,2E,6Z)-nona-2,6-dienal O-methyl oxime has a perception threshold of 2.94 ng/l. Similarly, whereas 3-(3-isopropylphenyl)butanal has a perception threshold of 0.07 ng/l, its corresponding oxime ether - E)-3-(3-isopropylphenyl)butanal O-methyl oxime - has a perception threshold of 22.4 ng/l.

Given that oxime ethers are generally much weaker than their corresponding fragrant aldehydes, it is perhaps unsurprising that they are not interesting to perfumers and there is no mention of perfume compositions containing them in the literature. The oxime ethers of the present invention are unusual in that they are uncharacteristically powerful (indeed, in the case of 3-ethoxy-4-hydroxybenzaldehyde O-methyl oxime it is comparable in strength as the powerful odourant ethyl vanillin).

Furthermore, applicant surprisingly found that whereas isoeugenol as well as certain isoeugenol "replacers" such as vanillin exhibit colour instability in consumer bases, the oxime ethers of formula (I) are colour stable. By the term "colour stable" is meant that when dosed in a colourless consumer base at 0.02 % and held under storage for 4 weeks at 45 °C, bases containing the compounds of formula (I) remain colourless upon visual inspection. On the other hand, bases containing isoeugenol and isoeugenol "replacers" such as vanillin at the same concentration were discoloured, variously appearing to have yellowish, orange, peachy or brown hues.

In a particular embodiment of the present invention there is provided a fragrance oil comprising a compound of formula (I) and additionally one or more perfume ingredients, more particularly the compound wherein R₁ represents ethyl and R₂ represent methyl group, and no iso-eugenol. In another aspect of the present invention there is provided a consumer product as defined in claim 7.

In yet another aspect of the present invention there is provided a consumer product comprising a fragrance oil as defined in claims 1-5, more particularly comprising the compound of formula (I) wherein R₁ represents ethyl and R₂ represent methyl group, and no iso-eugenol .

In yet another aspect of the present invention there is provided a consumer product, comprising a fragrance oil as defined in claims 1-5, and iso-eugenol, more particularly comprising the compound of formula (I) wherein R₁ represents ethyl and R₂ represent methyl group, wherein the level of iso-eugenol is less than 100 ppm.

Compounds of formula (I) due to their desirable odour profiles and/or their good colour stability and/or their low allergenic potential provide the perfumer the latitude to use them over wide-ranging amounts, and certainly with less restriction on their usage level than currently imposed on isoeugenol or indeed isoeugenol "replacers" such as vanillin or methyl diantilis.

In a particular embodiment of the present invention, the compounds of formula (I) may be employed at levels ranging from 0.1 % to 20 % by weight in a fragrance oil, corresponding to usage levels of 10 to 2000 ppm in a consumer product.

The compounds of formula (I) therefore, have much wider usage levels than isoeugenol, and can be used in much higher levels than isoeugenol in fragrance oils and across a wide range of consumer product categories.

In a particular embodiment of the present invention the compounds of formula (I) may be employed in a deodorant or antiperspirant formulation in an amount of 50 to 1000 ppm.

In a particular embodiment of the present invention the compounds of formula (I) may be employed in a rinse-off formulation such as shampoo and hair conditioner in an amount of 100 to 1000 ppm.

In a particular embodiment of the present invention the compounds of formula (I) may be employed in a fabric conditioner formulation in an amount of 50 to 1000 ppm.

In a particular embodiment of the present invention the compounds of formula (I) may be employed in a liquid detergent formulation in an amount of 10 to 1000 ppm.

In a particular embodiment of the present invention the compounds of formula (I) may be employed in a WC rim cleaner formulation in an amount of 10 to 1000 ppm.

In a particular embodiment of the present invention the compounds of formula (I) may be employed in an all purpose cleaner formulation in an amount of 10 to 1000 ppm.

The compounds of formula (I) were also surprisingly found to exhibit a very natural Ylang Ylang-type odour. The use of Ylang Ylang (cananga odorata) extracts is limited to 0.06 % by weight in IFRA category 2 products (anti-perspirant/deodorant compositions).

In such products in which an Ylang Ylang-type note is desired, it follows that compounds of formula (I) may be used as partial or complete replacers for Ylang Ylang extracts. The fragrance oil can comprise a compound of formula (I) and Ylang Ylang extract, more particularly the compound of formula (I) wherein R₁ represents ethyl and R₂ represent methyl group, wherein the level of Ylang Ylang extract is less than 1 % by weight, or even no Ylang Ylang extract is present. In another aspect of the present invention there is provided an antiperspirant or deodorant composition comprising a fragrance oil as defined in claims 1-5 and Ylang Ylang extract at a level of 600 ppm or less, more particularly comprising the compound of formula (I) wherein R₁ represents ethyl and R₂ represent methyl group.

In another aspect of the present invention there is provided an antiperspirant or deodorant composition comprising a fragrance oil as defined in claims 1-5, more particularly comprising the compound wherein R₁ represents ethyl and R₂ represent methyl group, and no Ylang Ylang extract. The applicant is not aware, however, of any single fragrance ingredient that has an odour profile that corresponds precisely to that of isoeugenol or ylang ylang. The compounds of formula (I) therefore possess an odour profile that is similar to, but not exactly the same as that of isoeugenol or ylang ylang. As such, perfumers may wish to mix the compounds of formula (I) with certain other perfume ingredients to provide fragrance oils having closer conformance to the odour profile of isoeugenol or ylang ylang, or even to achieve substantial equivalence to the odour profile of isoeugenol or ylang ylang. By "substantial equivalence" is meant that a panel of non-experts, e.g. consumers would be unable to detect a difference between a consumer product containing isoeugenol or ylang ylang and the same product containing a fragrance oil containing a compound of formula (I) of the present invention.

In a particular embodiment of the present invention a compound of formula (I) may form a fragrance oil in combination with one or more of the perfume ingredients selected from the group consisting of Agrumex, Allyl Amyl Glycolate, Ambrettolide, Ambrofix, Amyl Salicylate, Anisic Aldehyde, Benzyl Acetate, Benzyl Benzoate, Benzyl Salicylate, Bornyl Acetate, 4-t-Butyl Cyclohexyl Acetate, Cetalox, Citral, Citronellol, Citronellyl Acetate, Coumarin, Cyclamen Aldehyde, Alpha Damascone, Delta Damascone, Gamma Decalactone, Decanal, Dihydro Myrcenol, Diphenyl Oxide, Dodecanal, Ebanol, Ethyl Linalool, Ethyl Maltol, Ethyl 2-Methylbutyrate, Ethyl Vanillin, Ethylene Brassylate, Eugenol, Evernyl, Fixolide, Florocyclene, Galaxolide, Gardenol, Geraniol, Geranyl Acetate, Habanolide, Hedion, Heliotropine, Z-3-Hexenol, Z-3-Hexenyl Acetate, Z-3-Hexenyl Salicylate, Hexyl Acetate, Alpha Hexyl Cinnamic Aldehyde, Hexyl Salicylate, Indole, Alpha lonone, Beta lonone, Iso E Super, Isoraldeine, Isoeugenol, Jasmacyclene, Javanol, Lilial, Linalool, Linalyl Acetate, Longifolene, Maltol, Manzanate, 2-(1-Mercapto-1-methylethyl)-5-methyl Cyclohexanone, Methyl Benzoate, Methyl Diantilis, Methyl Napthyl Ketone, 2-Methyl Undecanal, Muscenone, Nectaryl, Peach Pure, Peonile, Petalia, Phenoxy Ethyl Isobutyrate, 2-Phenylethyl Acetate, Phenyl Acetaldehyde, 2-Phenyl Ethanol, Radjanol, Rosacetol, Spirogalbanone, Terpineol, Terpinolene, Terpinyl Acetate, Tetrahydro Linalool, 4,7,7-Trimethyl-6-thiabicyclo[3.2.1.]octane, Tricyclal, Tropional, 9-Undecenal, Undecavertol and Vanillin.

In a particular embodiment of the present invention, if a perfume is desired that may be useful to reproduce the character if ylang ylang a compound of the formula (I) may be combined with one or more ingredients selected from the group consisting of Amyl Salicylate, Benzyl Acetate, Benzyl Benzoate, Benzyl Salicylate, Citral, Citronellol, Citronellyl Acetate, para Cresyl Methyl Ether, Alpha Damascone, Delta Damascone, Decanal, Dodecanal, Ethyl Linalool, Ethyl 2-Methylbutyrate, Ethyl Vanillin, Eugenol, Geraniol, Geranyl Acetate, Habanolide, Hedione, Z-3-Hexenol, Z-3-Hexenyl Acetate, Z-3-Hexenyl Salicylate, Hexyl Acetate, Alpha Hexyl Cinnamic Aldehyde, Hexyl Salicylate, Indole, Alpha lonone, Beta lonone, Isoeugenol, Linalool, Linalyl Acetate, Manzanate, Methyl Benzoate, Methyl Diantilis, -2-Phenylethyl Acetate, Phenyl Acetaldehyde, 2-Phenyl Ethanol, Terpineol, Terpinolene, Terpinyl Acetate, Tetrahydro Linalool, Tricyclal and Vanillin.

Fragrance oils need not be limited to a mixture of those ingredients mentioned above, and other ingredients commonly used in perfumery may be employed, for example any of those ingredients described in "Perfume and Flavour Chemicals", S. Arctander, Allured Publishing Corporation, 1994, IL, USA. Fragrance oils of the present invention and consumer products containing same may also contain commonly employed adjuvants. The term "adjuvants" refers to ingredients that may affect the performance of a composition, other than its hedonic performance. For example, an adjuvant may be an ingredient that acts as an aid to processing a fragrance oil or consumer product containing said fragrance oil, or it may improve handling or storage of a fragrance oil or consumer product. It might also be an ingredient that provides additional benefits such as imparting colour or texture. It might also be an ingredient that imparts light resistance or chemical stability to one or more ingredients contained in a fragrance oil or consumer product. A detailed description of the nature and type of adjuvants commonly used in fragrance oils or consumer products cannot be exhaustive, but it has to be mentioned that said ingredients are well known to a person skilled in the art. Examples of adjuvants include solvents and co-solvents; surfactants and emulsifiers; viscosity and rheology modifiers; thickening and gelling agents; preservative materials; pigments, dyestuffs and colouring matters; extenders, fillers and reinforcing agents; stabilisers against the detrimental effects of heat and light, bulking agents, acidulants, buffering agents and antioxidants.

If the fragrance oils employ a mixture of ingredients to reproduce the character of isoeugenol or ylang ylang, these ingredients will all have different physical or chemical properties, such as volatility and substantivity. Accordingly, for the fragrance oils to retain a coherent character of isoeugenol or ylang ylang throughout its use cycle, it may be desirable to accelerate or retard the release of one or more ingredients depending on the particular end use. For example, when the fragrance oils are to be used in a laundry detergent composition, it might be desirable to formulate one or more non-substantive ingredients in a delivery vehicle that provides the ingredient(s) with the requisite substantivity such that the character of iso-eugenol or ylang ylang is evident through the whole use cycle from bottle to wash cycle to dry down.

Alternatively, in an end use application where a perfume is expected to bloom upon dilution, such as in the case of a hard-surface cleaner, it will be desirable to formulate the ingredients in such a manner that the individual ingredients develop at substantially the same rate during the cleaning period.

Accordingly, one or more of the ingredients employed in said fragrance oils or consumer products may be formulated in a delivery vehicle to provide a desired effect. Delivery vehicles may include capsule technologies. Alternatively, the delivery vehicle may be in the form of a solid support, e.g. a polymeric support material onto which one or more perfume ingredients may be chemically or physically bound. Still further, one or more perfume ingredients may be dissolved or dispersed in a matrix material, which serves to control the rate at which said ingredient or ingredients emanates. In yet an alternative embodiment, one or more ingredients may be supported on a porous substrate, such as a cyclodextrin or a zeolite or other inorganic material. In a still further embodiment, one or more ingredients may be provided in the form of a pro-perfume, which will react in a suitable environment to release the perfume ingredient in a controlled manner. The fragrance oils may be employed in all manner of personal and home care consumer products set out in the IFRA categories. A non-limiting list of applications include a textile treatment product, an ironing aid, a cleaning cloth, a laundry detergent, a cleaning product, in particular, for hard and/or soft surfaces, a household cleaner, a care product, a wash care product, a laundry care product, a room fragrancer, and air freshener, a conditioner, a colorant, a fabric conditioner, a conditioning substrate, a pharmaceutical, a crop protection product, a polish, a food, a cosmetic product, a fertilizer, a building material, an adhesive, a bleach, a decalcifier, an autocare product, floorcare product, cookercare product, leather care product or furniture care product, a scourer, a disinfectant, a fragrancer, a mould remover, fine fragrance, body lotion, skin care preparations, candles, air fresheners, plug ins and toilet soaps.

Particular examples of cleaning products include the toilet cleaners or lavatory cleaners, these products being supplied in the form of powders, blocks, tablets or liquids, or gels, pipe-cleaning products or drain cleaners, universal or all-purpose or general-purpose cleaners, such as those used universally for all hard surfaces in the household and in commerce that can be wiped down wet or damp, sanitary cleaners, oven cleaners or grill cleaners which may be presented in the form of gels or foam sprays, metal polishes, including those supplied as polishing cloths, dipping baths, pastes, and liquids; glass cleaners and window cleaners; all special-purpose cleaning products, for example those for glass-ceramic hobs; carpet cleaners and stain removers.

Particular examples of auto care products include paint preservers, paint polishes, paint cleaners, wash preservers, shampoos for auto washing, auto-wash and wax products, polishes for trim metals, protective films for trim metals, plastics cleaners, tar removers, screen cleaners, engine cleaners and the like.

Particular examples of cosmetic products include cosmetic skincare products, e.g. bath products, skin washing and cleansing products, skincare products, eye makeup, lip care products, nail care products, intimate care products, foot care products; cosmetic products with specific effects, such as sunscreens, tanning products, de-pigmenting products, deodorants, antiperspirants, hair removers, shaving products; cosmetic dental-care products, such as dental and oral care products, toothcare products, cleaners for dental prostheses, adhesives for dental prostheses; cosmetic hair care products, e.g. hair shampoos, hair care products, hair setting products, hair-shaping products, and hair colouring products.

Particular examples of textile treatment products include detergents or fabric conditioners.

Particular examples of air fresheners and room fragrancers include fragrancers for spaces such as autos, cupboards, dishwashers, refrigerators or shoes, and vacuum cleaners.

There now follows a series of examples that serve to illustrate the invention.

### Example 1

### Example: Compounds tested in the KeratinoSens™ assay for skin sensitization

The KeratinoSens™ cell line contains a stable insertion of a Luciferase gene under the control of the ARE-element of the gene AKR1C2. The optimization of this cell line and the standard method to test chemicals has been described in detail in Emter et. al. 2010 (Emter R., Ellis G., Natsch A., 2010 "Performance of a novel keratinocyte-based reporter cell line to screen skin sensitizers in vitro. Toxicol Appl Pharmacol 245, 281-290). Cells were grown for 24 h in 96-well plates. The medium was then replaced with medium containing the test chemical and the solvent Dimethylsulfoxide (DMSO) at a final level of 1%. Each compound was tested at 12 binary dilutions in the range from 0.98 to 2000 µM. In each repetition, three parallel replicate plates were run with this same set-up and a fourth parallel plate was prepared for cytotoxicity determination using an MTT assay. Cells were incubated for 48 h with the test agents, and then luciferase activity and cytotoxicity were determined. This full procedure was repeated at least three times for each chemical. For each chemical in each repetition and at each concentration, the gene induction compared to DMSO controls and the wells with statistically significant induction over the threshold of 1.5 (i.e. 50% enhanced gene activity) were determined. Furthermore the maximal fold-induction (Iₘₐₓ) and the EC1.5 value (concentration in µM for induction above the threshold) were calculated. Chemicals are rated as positive (i.e. likely skin-sensitizers) in the assay if the following three criteria are fulfilled (i) The EC1.5 value is below 1000 µM in all three repetitions or in at least 2 repetitions, (ii) at the lowest concentration with a gene induction above 1.5 fold (i.e. at the EC 1.5 determining value), the cellular viability is above 70% and (iii) there is an apparent overall dose-response for luciferase induction, which is similar between the repetitions.

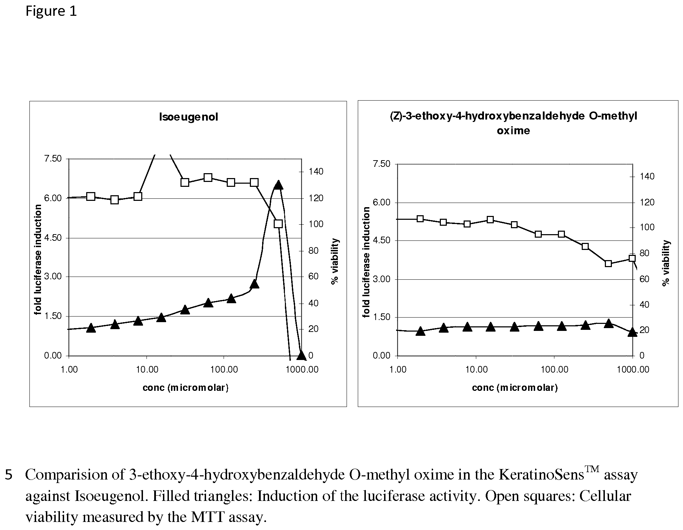

Figure 1 shows the gene-induction and cell viability curves for Isoeugenol and ethoxy-4-hydroxybenzaldehyde O-methyl oxime (compound of formula (I) wherein R₁ is ethyl and R₂ is methyl). The latter did not induce luciferase activity above the 1.5-fold threshold and is thus rated as non-sensitizing by this assay. It also has no significant cytotoxicity up to 1000 micromolar. Isoeugenol on the other hand clearly induces the gene already in the lower micromolar range, indicating it is a sensitizing compound. In addition at concentrations above 500 micromolar it is cytotoxic. These results show that 3-ethoxy-4-hydroxybenzaldehyde O-methyl oxime can be used in perfume formulations instead of Isoeugenol to reduce the sensitization risk to the consumer.

### Example 2: colour stability in consumer products

### Example 2a: Colour stability in fine fragrance

A solution of the test compound (compound of formula (I) specified in the Table below) was prepared in perfumery grade ethanol (96% EtOH, 4% water) at 1 and 0.5 % wt/wt. The solutions were placed in 2.5 ml transparent glass vials closed with plastic lids. The vials were placed behind a window exposed to indoor daylight. The colour of the solutions was inspected visually over a 1 month period.

| Test compound | Colour of ethanol solution after 1 month | Odour |
|---|---|---|
| Vanillin | yellow | vanilla |
| Ethyl Vanillin | yellow | vanilla |
| Cpd I (R₁= Et, R₂=Me) | colourless | Spicy vanilla floral |

The ethanol solutions perfumed with compound I of the present invention would have passed the requirements for colourless fine fragrance at both concentrations after 1 month, whereas the ones containing vanillin or ethyl vanillin had developed an unacceptable yellow colour.

### Example 2b: Colour stability in bar soap

A standard tallow toilet soap sample was prepared by adding 2% wt/wt of a 10% wt/wt solution in dipropylene glycol (DPG) of either ethyl vanillin or compound I (R₁= Et, R₁=Me) to the soap base. The extruded 24x8mm soap string was cut into 8 cm pieces, which were placed in a thermostated oven for 1 month at 45°C. A control sample containing no fragrance was added to the stability test series. After one month, the colour of the ethyl vanillin containing soap had turned into a chocolate brown, the odour was vanilla like. The colour of the soap containing compound I remained as control, its odour was vanilla spicy floral like.

## Claims

1. A fragrance oil comprising a compound of formula (I) 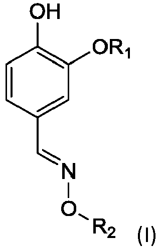 wherein R₁ represents a methyl group or an ethyl group and K₂ represents a methyl or an ethyl group, additionally comprising one or more perfume ingredients wherein the fragrance oil contains less than 1% by weight of isoeugenol.

2. A fragrance oil comprising a compound of formula (I) wherein R₁ is ethyl and R₂ represents a methyl group.

3. A fragrance oil according to claim 1 or claim 2 that contains no isoeugenol.

4. A fragrance oil according to any of the preceding claims wherein a compound of the formula (I) is employed at a level ranging from 0.1 % to 20 % by weight.

5. A fragrance oil according to any of the preceding claims, comprising one or more perfume ingredients selected from the group consisting of Agrumex, Allyl Amyl Glycolate, Ambrofix, Amyl Salicylate, Anisic Aldehyde, Benzyl Acetate, Benzyl Benzoate, Benzyl Salicylate, Bornyl Acetate, 4-t-Butyl Cyclohexyl Acetate, Cetalox, Citral, Citronellol, Citronellyl Acetate, Coumarin, Cyclamen Aldehyde, Alpha Damascone, g-Decalactone, Decanal, Dihydro Myrcenol, Diphenyl Oxide, Dodecanal, Ethyl Linalool, Ethyl 2-Methylbutyrate, Ethyl Vanillin, Ethylene Brassylate, Eugenol, Evernyl, Fixolide, Florocyclene, Galaxolide, Gardenol, Geraniol, Geranyl Acetate, Habanolide, Hedion, Heliotropine, Z-3-Hexenol, Z-3-Hexenyl Acetate, Hexyl Acetate, a-Hexyl Cinnamic Aldehyde, Hexyl Salicylate, Indole, b-lonone, Iso E Super, Isoraldeine 70, Isoeugenol, Jasmacyclene, Lilial, Linalool, Linalyl Acetate, Longifolene, Methyl Diantilis, 2-Methyl Undecanal, Muscenone, Peach Pure, Peonile, Phenoxy Ethyl Isobutyrate, 2-Phenylethyl Acetate, 2-Phenyl Ethanol, Rosacetol, Terpineol, Terpinolene, Terpinyl Acetate, Tetrahydro Linalool, Tricyclal, Tropional, Undecavertol and Vanillin.

6. A consumer product comprising a fragrance oil as defined in any of the preceding claims.

7. A consumer product according to claim 6 in the form of a deodorant or antiperspirant formulation wherein a compound of formula (I) is present in an amount of 50 to 1000 ppm.

8. A consumer product according to claim 6 in the form of a rinse-off formulation such as shampoo and hair conditioner wherein a compound of formula (I) is present in an amount of 100 to 1000 ppm.

9. A consumer product according to claim 6 in the form of a fabric care formulation such as fabric conditioner or tumble dryer sheets wherein a compound of formula (I) is present in an amount of 100 to 1000 ppm.

10. A consumer product according to claim 6 in the form of a liquid detergent formulation wherein a compound of formula (I) is present in an amount of 10 to 1000 ppm.

11. A consumer product according to claim 6 in the form of a WC rim cleaner formulation wherein a compound of formula (I) is present in an amount of 10 to 1000 ppm.

12. A consumer product according to claim 6 in the form of an all purpose cleaner formulation wherein a compound of formula (I) is present in an amount of 10 to 1000 ppm.

13. Use of the fragrance oil according to one of claims 1 to 5 as a replacement for isoeugenol.

## Patentansprüche

1. Duftöl, umfassend eine Verbindung der Formel (I) 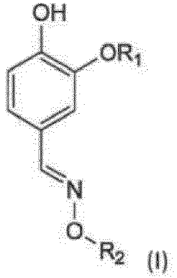 wobei R₁ eine Methylgruppe oder eine Ethylgruppe darstellt und R₂ eine Methyl- oder eine Ethylgruppe darstellt, zusätzlich umfassend einen oder mehrere Parfümbestandteile, wobei das Duftöl weniger als 1 Gew.-% Isoeugenol enthält.

2. Duftöl, umfassend eine Verbindung der Formel (I), wobei R₁ Ethyl ist und R₂ eine Methylgruppe darstellt.

3. Duftöl gemäß Anspruch 1 oder Anspruch 2, das kein Isoeugenol enthält.

4. Duftöl gemäß einem der vorstehenden Ansprüche, wobei eine Verbindung der Formel (I) in einer Menge in dem Bereich von 0,1 Gew.-% bis 20 Gew.-% eingesetzt wird.

5. Duftöl gemäß einem der vorstehenden Ansprüche, umfassend einen oder mehrere Parfümbestandteile ausgewählt aus der Gruppe bestehend aus Agrumex, Allylamylglycolat, Ambrofix, Amylsalicylat, Anisaldehyd, Benzylacetat, Benzylbenzoat, Benzylsalicylat, Bornylacetat, 4-t-Butylcyclohexylacetat, Cetalox, Citral, Citronellol, Citronellylacetat, Coumarin, Cyclamenaldehyd, alpha-Damascon, g-Decalacton, Decanal, Dihydromyrcenol, Diphenyloxid, Dodecanal, Ethyllinalool, Ethyl-2-methylbutyrat, Ethylvanillin, Ethylenebrassylat, Eugenol, Evernyl, Fixolid, Florocyclen, Galaxolid, Gardenol, Geraniol, Geranylacetat, Habanolid, Hedion, Heliotropin, Z-3-Hexenol, Z-3-Hexenylacetat, Hexylacetat, a-Hexylzimtsäurealdehyd, Hexylsalicylat, Indol, b-Ionon, Iso E Super, Isoraldein 70, Isoeugenol, Jasmacyclen, Lilial, Linalool, Linalylacetat, Longifolen, Methyldiantilis, 2-Methylundecanal, Muscenon, Peach Pure, Peonil, Phenoxyethylisobutyrat, 2-Phenylethylacetat, 2-Phenylethanol, Rosacetol, Terpineol, Terpinolen, Terpinylacetat, Tetrahydrolinalool, Tricyclal, Tropional, Undecavertol und Vanillin.

6. Verbraucherprodukt, umfassend ein Duftöl gemäß einem der vorstehenden Ansprüche.

7. Verbraucherprodukt gemäß Anspruch 6 in der Form einer Deodorant- oder Antiperspirant-Formulierung, worin eine Verbindung der Formel (I) in einer Menge von 50 bis 1000 ppm vorhanden ist.

8. Verbraucherprodukt gemäß Anspruch 6 in der Form einer ausspülbaren Formulierung, wie z. B. Shampoo oder Haarkonditionierungsmittel, worin eine Verbindung der Formel (I) in einer Menge von 100 bis 1000 ppm vorhanden ist.

9. Verbraucherprodukt gemäß Anspruch 6 in der Form einer Textilpflegeformulierung, wie z. B. Textilkonditionierungsmittel oder Trommeltrocknertücher, worin eine Verbindung der Formel (I) in einer Menge von 100 bis 1000 ppm vorhanden ist.

10. Verbraucherprodukt gemäß Anspruch 6 in der Form einer flüssigen Detergensformulierung, worin eine Verbindung der Formel (I) in einer Menge von 10 bis 1000 ppm vorhanden ist.

11. Verbraucherprodukt gemäß Anspruch 6 in der Form einer WC-Rand-Reinigungsformulierung, worin eine Verbindung der Formel (I) in einer Menge von 10 bis 1000 ppm vorhanden ist.

12. Verbraucherprodukt gemäß Anspruch 6 in der Form einer Allzweckreinigerformulierung, worin eine Verbindung der Formel (I) in einer Menge von 10 bis 1000 ppm vorhanden ist.

13. Verwendung des Duftöls gemäß einem der Ansprüche 1 bis 5 als Ersatz für Isoeugenol.

## Revendications

1. Huile parfumée, comprenant un composé de formule (I) 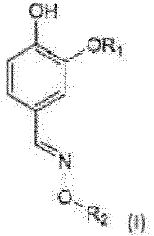 où R₁ représente un groupement méthyle ou un groupement éthyle et R₂ représente un groupement méthyle ou éthyle, comprenant en outre un ou plusieurs ingrédients de parfum, où l'huile parfumée contient moins de 1% en poids d'isoeugénol.

2. Huile parfumée, comprenant un composé de formule (I), où R₁ est éthyle et R₂ représente un groupement méthyle.

3. Huile parfumée selon la revendication 1 ou la revendication 2, qui ne contient pas d'isoeugénol.

4. Huile parfumée selon l'une quelconque des revendications précédentes, où un composé de formule (I) est employé à un taux allant de 0,1% à 20% en poids.

5. Huile parfumée selon l'une quelconque des revendications précédentes, comprenant un ou plusieurs ingrédients de parfum choisis dans le groupe constitué par l'Agrumex, le Glycolate d'allyl-amyle, l'Ambrofix, le Salicylate d'amyle, l'Aldéhyde anisique, l'Acétate de benzyle, le Benzoate de benzyle, le Salicylate de benzyle, l'Acétate de bornyle, l'Acétate de 4-t-butyl-cyclohexyle, le Cétalox, le Citral, le Citronellol, l'Acétate de citronellyle, la Coumarine, l'Aldéhyde de cyclamen, l'alpha-Damascone, la g-Décalactone, le Décanal, le Dihydro-myrcénol, l'Oxyde de diphényle, le Dodécanal, le Linalool d'éthyle, le 2-Méthylbutyrate d'éthyle, la Vanilline d'éthyle, le Brassylate d'éthylène, l'Eugénol, l'Evernyl, le Fixolide, le Florocyclène, le Galaxolide, le Gardénol, le Géraniol, l'Acétate de géranyle, l'Habanolide, l'Hédione, l'Héliotropine, le Z-3-Hexénol, l'Acétate de Z-3-hexényle, l'Acétate d'hexyle, l'Aldéhyde a-hexyl-cinnamique, le Salicylate d'hexyle, l'Indole, la b-Ionone, l'Iso E Super, l'Isoraldéine 70, l'Isoeugénol, le Jasmacyclène, le Lilial, le Linalool, l'Acétate de linalyle, le Longifolène, le Méthyl Diantilis, le 2-Méthyl-undécanal, la Muscénone, le Peach Pure, le Péonile, l'Isobutyrate de phénoxyéthyle, l'Acétate de 2-phényléthyle, le 2-Phényléthanol, le Rosacétol, le Terpinéol, le Terpinolène, l'Acétate de terpinyle, le Tétrahydrolinalool, le Tricyclal, le Tropional, l'Undécavertol et la Vanilline.

6. Produit de consommation comprenant une huile parfumée telle que définie selon l'une quelconque des revendications précédentes.

7. Produit de consommation selon la revendication 6, sous la forme d'une formulation de déodorant ou d'antitranspirant, où un composé de formule (I) est présent selon une quantité allant de 50 à 1000 ppm.

8. Produit de consommation selon la revendication 6, sous la forme d'une formulation à rincer telle qu'un shampooing ou un après-shampooing, où un composé de formule (I) est présent selon une quantité allant de 100 à 1000 ppm.

9. Produit de consommation selon la revendication 6, sous la forme d'une formulation de soin du linge telle qu'un assouplissant ou des lingettes pour sèche-linge, où un composé de formule (I) est présent selon une quantité allant de 100 à 1000 ppm.

10. Produit de consommation selon la revendication 6, sous la forme d'une formulation détergente liquide, où un composé de formule (I) est présent selon une quantité allant de 10 à 1000 ppm.

11. Produit de consommation selon la revendication 6, sous la forme d'une formulation de nettoyage des rebords de cuvette de WC, où un composé de formule (I) est présent selon une quantité allant de 10 à 1000 ppm.

12. Produit de consommation selon la revendication 6, sous la forme d'une formulation nettoyante tous usages, où un composé de formule (I) est présent selon une quantité allant de 10 à 1000 ppm.

13. Utilisation de l'huile parfumée selon l'une des revendications 1 à 5, comme remplacement de l'isoeugénol.
